# EUROPEAN PATENT APPLICATION

(11) **EP 4 506 496 A1**
(43) Date of publication of application: **12.02.2025**
(21) Application number: 22935859.3
(22) Date of filing: 31.05.2022
(51) Int. Cl.: D04H 1/4266, D04H 1/48, A45D 44/00, A61L 15/28, A61L 31/04, D04H 1/728, D04H 1/485

(54) **NON-WOVEN FABRIC USING HYALURONATE FIBER AND METHOD FOR MANUFACTURING SAME**

(30) Priority: 31.03.2022 KR 20220040033; 23.05.2022 KR 20220062829
(71) Applicant: Jinwoo Bio Co., Ltd., Giheung-gu Yongin-si, Gyeonggi-do 17111 (KR)
(72) Inventor: KWEON, Dong Keon, Yongin-si Gyeonggi-do 16918 (KR); LEE, Myoung Han, Seoul 02799 (KR); KIM, Jong Soo, Bucheon-si Gyeonggi-do 14539 (KR); HONG, Joo Yeon, Guri-si Gyeonggi-do 11920 (KR)
(74) Representative: Ter Meer Steinmeister & Partner
(86) International application number: PCT/KR2022/007708
(87) International publication number: WO 2023/191184

(57) **Abstract**

The present invention relates to a hyaluronate non-woven fabric made of hyaluronate fibers and a method of manufacturing the same. More particularly, the present invention relates to a highly functional hyaluronate non-woven fabric made of hyaluronate fibers having a rough surface and a micro-size diameter and a method of manufacturing the same. A hyaluronate non-woven fabric manufacturing method according to the present invention includes (a) preparing hyaluronate fibers, (b) treating the hyaluronate fibers to be curled, (c) cutting the curled hyaluronate fibers, (d) forming a hyaluronate web from the curled hyaluronate fibers, and (e) needle-punching the hyaluronate web. The hyaluronate non-woven fabric of the present invention has a high hyaluronate content, is manufactured by needle punching high-strength hyaluronate fibers, and has excellent adhesion and tensile strength. Therefore, the hyaluronate non-woven fabric of the present invention can be used as a mask pack for external application on the skin, a biomaterial for tissue repairing, and an anti-adhesion coating material.

## Description

### Technical Field

The present invention relates to a hyaluronate non-woven fabric made of hyaluronate fibers and a method of manufacturing the same. More particularly, the present invention relates to a highly functional hyaluronate non-woven fabric made of hyaluronate fibers having a rough surface and a micro-size diameter and a method of manufacturing the same.

### Background Art

Non-woven fabric is a flat fibrous structure made by forming sheet-shaped webs in which various fibers such as natural fibers, chemical fibers, glass fibers, and metal fibers are tangled according to their characteristics and then mechanically or physically combining the webs. Such non-woven fabric is widely and diversely used in various industrial fields according to its use, characteristics, and functions. In addition, research has been conducted on developing various non-woven fabrics by performing secondary processing on existing non-woven fabrics or adding components capable of imparting various functions to the existing non-woven fabrics.

In recent years, the consumption of products utilizing non-woven fabrics has been increasing day by day in several industries. For example, non-woven fabric is hot-pressed to produce a hard panel to be used as a finishing material for automobiles or buildings. In a wide variety of fields, such as clothing, sanitation, civil engineering, construction, automobiles, and medical, intensive research is being conducted on value-added non-woven fabric products that are advanced products from existing simple products and which have various functions. In particular, in the medical field, non-woven fabric products are not only used for wearable products such as surgical gowns and masks but also as surgical drapes, pads, dressings, and filter materials, and even as tissue supports to be inserted into the body for regeneration of internal organs.

To prepare these medical-purpose non-woven fabrics, various biodegradable polymers are used. Biodegradable polymers are polymers that gradually lose their shape and weight as they undergo chemical degradation in vivo. Examples of natural polymers include collagen, fibronectin, gelatin, chitosan, and alginic acid, and examples of synthetic polymers include poly(lactic acid) (PLA), poly(glycolic acid) (PGA), poly(D,L-lactic-co-glycolic acid) (PLGA) and similar copolymers, poly(ε-caprolactone) (PCL), polyanhydrides, and polyorthoesters.

Korean Patent No. 1329261 discloses functional non-woven fabric using chitosan and a manufacturing method thereof, and Korean Patent No. 2002586 discloses thermally bonded non-woven fabric containing natural fibers having a form in which chitosan is coated on cellulose fibers.

Hyaluronic acid (HA), on the other hand, is a mucopolysaccharide present in the dermis layer and is a biopolymer in which disaccharides formed by β-1,4 glucosidic bonding of N-acetyl glucosamine and D-glucuronic acid are linked by β-1,3 glucosidic bonds to form hyaluronate chains.

Hyaluronic acid is evenly distributed throughout the connective, epithelial, and neural tissues of the human body and is a biocompatible material with various physiological activities. As it has been proven to be effective in skin regeneration, moisturizing, elasticity maintenance, and wrinkle improvement, it has recently been necessary for the industry to manufacture non-woven fabrics from hyaluronate acid fibers for use as wound coverings, beauty patches, and anti-adhesion coverings. However, hyaluronate fibers to be used as raw materials for non-woven fabrics have problems described below, depending on the manufacturing methods thereof.

Hyaluronic acid nanofiber manufacturing methods involve the process of removing organic solvents such as DMF, DMSO, and formic acid, which are essential volatile required for the manufacture of nanofibers. When manufacturing non-woven fabrics from the hyaluronic acid nanofibers, it is inconvenient to use nanofibers alone, and there is a disadvantage that the nanofibers have poor physical properties. In addition, there is a problem in that the production unit cost is high due to very low productivity per unit time.

To solve these problems, Korean Patent No. 2338355 discloses a wet non-woven fabric manufacturing method, but the non-woven fabric manufactured by the method has poor physical properties, and the procedures of the manufacturing method are somewhat complicated. Therefore, research is needed for the development of more efficient manufacturing methods. When it is used in the form of non-woven fabric, if the non-woven fabric does not have a certain level of strength, it is difficult to commercialize the non-woven fabric because processing operations such as spinning for commercialization cannot be performed.

Accordingly, the present inventors have made efforts to solve the problems and found that when a hyaluronate paste with a moisture content of 50 wt% to 95 wt% is prepared, melted, extruded through a nozzle, and then dried, hyaluronate fibers with a rough surface and excellent tensile strength can be produced in large quantities, and hyaluronate non-woven fabrics with good adhesion and tensile strength can be produced from the fibers through needle punching. Thus, the present invention could be made.

### Disclosure

### Technical Problem

It is an objective of the present invention to provide a hyaluronate non-woven fabric which not only can be easily adhered when utilized on the skin or in the body but also has excellent mechanical properties which are required for processing thereof, so that the hyaluronate non-woven fabric can be effectively used for mask packs for external application to the skin, a biomaterial for tissue repairing, or an anti-adhesion covering material. It is another objective of the present invention is to provide a method of manufacturing the hyaluronate non-woven fabric.

### Technical Solution

To achieve the above objectives, the present invention provides a hyaluronate non-woven fabric including hyaluronate fibers having a bending angle of 2° to 10°, a moisture content of 5 to 25 wt%, and a hyaluronate content of 90 wt% or more with respect to a total fiber weight excluding a weight of moisture.

### <Bending Angle Measurement Method>

(a) Prepare HA fibers with a length of 20 cm and a diameter of 1 mm
(b) Fix a first end of each HA fiber by a length of 3 cm on an upper surface of a flat plate
(c) Measure an angle (θ) between an extension line (T) from a second length (remaining end) of each HA fiber and an extension line (B) of the upper surface of the flat plate

In the present invention, the hyaluronate fiber may have a tensile strength in a range of 3 to 15 kg/cm².

In the present invention, the hyaluronate non-woven fabric may have a adhesion strength in a range of 0.9 to 2.7 kPa.

In the present invention, the hyaluronate non-woven fabric may have a thickness in a range of 1 to 10 mm.

In the present invention, the hyaluronate non-woven fabric may have a tensile strength in a range of 0.3 to 1.5 kg/cm².

In the present invention, the hyaluronate non-woven fabric is used as a mask pack for external application to the skin, a biomaterial for tissue repairing, or an anti-adhesion coating material.

In addition, the present invention provides a method of manufacturing a hyaluronate non-woven fabric, the method including: (a) preparing hyaluronate fibers; (b) treating the hyaluronate fibers to be curled; (c) cutting the curled hyaluronate fibers; (d) forming a hyaluronate web from the curled hyaluronate fibers; and (e) needle-punching the hyaluronate web.

In the present invention, the preparing of the hyaluronate fibers includes (a-1) preparing a hyaluronate paste by adjusting the moisture content of a hyaluronate having a weight-average molecular weight of 100 to 3,000 kDa to a range of 50 to 95 wt%; (a-2) melting the moisture-controlled hyaluronate paste at a temperature of 15°C to 100°C, and then extruding a fluid resulting from the melting of the hyaluronate paste through a nozzle; and (a-3) drying the extruded spun fluid to form a fiber.

In the present invention, when the web is formed, fibers made of a polymeric material selected from the group consisting of collagen, fibrin, gelatin, chitosan, alginic acid, polylactic acid (PLA), polyglutamic acid (PGA), polylactide-glycolide copolymer (PLGA), and polycaprolactone (PCL) is mixed.

### Advantageous Effects

The hyaluronate non-woven fabric of the present invention contains hyaluronate in a large amount, is manufactured by needle punching high strength hyaluronate fibers, and has excellent adhesion and tensile strength.

### Description of Drawings

FIG. 1 is a photograph showing the roughness of a hyaluronate fiber according to one embodiment of the present invention. (A: smooth hyaluronate fiber, B: rough hyaluronate fiber);
FIG. 2 is a diagram illustrating a method of measuring a bending angle of a hyaluronate fiber according to one embodiment of the present invention;
FIG. 3 is a photograph of a hyaluronate fiber according to one embodiment of the present invention. (A: hyaluronate fiber, B: curled hyaluronate fiber);
FIG. 4 is a photograph of a hyaluronate non-woven fabric manufactured through needle punching, according to one embodiment of the present invention;
FIG. 5 is a photograph of a hyaluronate and chitosan blended non-woven fabric according to one embodiment of the present invention; and
FIG. 6 is a photograph showing a microstructure of a needle punching non-woven fabric of the present invention. (A: hyaluronate non-woven fabric, B: hyaluronate and chitosan blended non-woven fabric)

### Best Mode

In the present invention, when a hyaluronate paste having a moisture content controlled to be in a specific range is dried at a high temperature rather than room temperature while being spun through a nozzle at a predetermined temperature, a hyaluronate fiber with a rough fiber surface can be manufactured, and a non-woven fabric with excellent adhesion and mechanical strength can be manufactured by needle-punching the hyaluronate fibers.

In the present invention, the moisture content of hyaluronate is adjusted to 50 to 95 wt% to produce a hyaluronate paste. The temperature of the hyaluronate paste is heated to a temperature range of 15°C to 100°C so as to be melted, then extruded through a nozzle, and then dried at a temperature in a range of 100°C to 150°C. Thus, hyaluronate fibers having a moisture content of 5 to 25 wt%, a tensile strength of 3 to 15 kg/cm², and a rough surface are manufactured in large quantities. Heat is applied to the hyaluronate fibers so that the hyaluronate fibers may be curled, the curled hyaluronate fibers are cut to have a predetermined length, the cut fibers are subjected to carding and air-laying to produce a web, and the webs are needle-punched to produce a piece of non-woven fabric, and the piece of non-woven fabric is pressed to produce non-woven fabric with excellent adhesion and tensile strength.

In addition, the present invention relates to a method of manufacturing a hyaluronate non-woven fabric, the method including: (a) preparing hyaluronate fibers; (b) treating the hyaluronate fibers to be curled; (c) cutting the curled hyaluronate fibers; (d) forming a hyaluronate web from the curled hyaluronate fibers; and (e) needle-punching the hyaluronate web.

In the present invention, the preparing of the hyaluronate fibers includes (a-1) preparing a hyaluronate paste by adjusting the moisture content of a hyaluronate having a weight-average molecular weight of 100 to 3,000 kDa to a range of 50 to 95 wt%; (a-2) heating the moisture-controlled hyaluronate paste to a temperature range of 15°C to 100°C so that the moisture-controlled hyaluronate paste can be melted, and then extruding a fluid resulting from the melting of the hyaluronate paste through a nozzle; and (a-3) drying the extruded spun fluid to form hyaluronate fibers.

In the present invention, the hyaluronate is a combined form of a salt and hyaluronic acid. Examples of the hyaluronate include, but are not limited to, sodium hyaluronate, calcium hyaluronate, potassium hyaluronate, and the like.

In the present invention, the molecular weight of the hyaluronate is preferably in a range of 100 to 3,000 kDa. In addition, the content of the hyaluronate in the hyaluronate paste is in a range of 50 to 95 wt%.

The hyaluronate paste may be prepared by applying a solvent to the hyaluronate. The solvent may be water or an aqueous solution of ethanol. Preferably, the solvent may be mixed for uniform mixing.

To facilitate spinning in a large volume, the moisture content of the hyaluronate paste may be adjusted to 50 to 95 wt% and preferably to 88 to 92 wt%. When the moisture content is lower than 50 wt%, the pressure for spinning the paste needs to be high. When the moisture content is higher than 95 wt%, the hyaluronate is present in the form of a liquid phase rather than a paste phase. In this case, there is a problem that threads easily break during spinning.

In the present invention, after the preparation of the hyaluronate paste, the hyaluronate paste is allowed to stand at a temperature of 0°C to 100°C for 6 to 12 hours for moisture equilibration. When the temperature or the duration is out of the corresponding range, the moisture equilibrium is not properly reached. The water equilibration is to ensure that water molecules and hyaluronic acid in the paste are evenly mixed. When spinning is performed without the moisture equilibration, water molecules and hyaluronic acid are not evenly mixed, and thus spinning cannot be performed at a constant speed.

In the present invention, the hyaluronate paste having a controlled moisture content of 50 to 95 wt% is placed in a spinning device, melted at a temperature in a range of 15°C to 100°C, and then extruded through a nozzle to produce a spinning fluid. In this case, when the temperature is lower than 15°C, the hyaluronate paste does not melt and thus cannot be extruded. When the temperature exceeds 100°C, the melt boils.

In the present invention, the extruded spinning fluid is dried to form a fiber. Preferably, the drying is performed at a temperature in a range of 100°C to 150°C depending on the moisture content of the paste. When the drying temperature is lower than 100°C, since the surface of the manufactured fiber is smooth, the tangling is not good during the needle punching process after the web formation. That is, it is difficult to form a non-woven fabric. When the drying temperature exceeds 150°C, there are problems in that the fiber is browned and a crack occurs.

The prepared hyaluronate fibers may have a hyaluronate content of more than 90 wt%. The hyaluronate fibers may have a moisture content of 5 wt% to 25 wt%, a tensile strength of 3 to 15 kg/cm², and an extremely rough surface. Therefore, when the hyaluronate fibers are used to form non-woven fabrics, non-woven fabrics having various densities and physical properties can be manufactured.

The method of manufacturing a hyaluronate non-woven fabric according to the present invention is characterized in that the fibers are curled by heat treatment to increase the tangling force of the fibers. In the case of heat treatment, the hyaluronate fibers may be treated by hot air at a temperature of 70°C or higher, so that the hyaluronate fibers can be curled.

To form a web for manufacturing a non-woven fabric, the web can be formed by carding and air-laying processes in which the manufactured hyaluronate fibers are cut to fiber pieces having the desired length, and the cut hyaluronate fibers are dispersed by air pressure and then injected by air pressure.

The needle punching step of the present invention is a step of punching sheet-shaped webs using a needle. Specifically, the needle punching step is a process of repeatedly moving a needled bed up and down as the webs pass through a needle loom, thereby combining portions of each of the two-dimensional random fiber arrays into a three-dimensional random structure.

The hyaluronate non-woven fabric manufacturing method of the present invention may include a step of adding and mixing fibers made of a biocompatible material when forming a web. That is, collagen, fibrin, gelatin, chitosan, alginic acid, polylactic acid (PLA), polyglutamic acid (PGA), polylactide-glycolide copolymers (PLGA), polycaprolactone (PCL), and the like that are pharmacologically acceptable biocompatible materials and which can be made into fibers may be blended with the hyaluronate fibers to impart a wider range of properties and functions to the hyaluronate fibers.

In another aspect, the present invention relates to a hyaluronate non-woven fabric including hyaluronate fibers, the fabric having a bending angle of 2° to 10°, a moisture content of 5 to 25 wt%, and a hyaluronate content of 90 wt% or more with respect to a total fiber weight excluding a weight of moisture, as measured by the following method.

### <Bending Angle Measurement Method>

(a) Prepare HA fibers with a length of 20 cm and a diameter of 1 mm
(b) Fix a first end of each HA fiber by a length of 3 cm on an upper surface of a flat plate
(c) Measure an angle (θ) between an extension line (T) from a second length (remaining end) of each HA fiber and an extension line (B) of the upper surface of the flat plate

Since the tensile strength of the hyaluronate fibers is in a range of 3 to 15 kg/cm², non-woven fabrics can be manufactured through needle punching. When the tensile strength of the hyaluronate fibers is less than 3 kg/cm², the fibers are highly likely to break, and thus it is difficult to process the fibers. When the tensile strength exceeds 15 kg/cm², the material is hard, which can cause discomfort in use after being made into non-woven fabric.

Preferably, the hyaluronate non-woven fabric of the present invention has a thickness of 1 to 10 mm. The manufactured non-woven fabric has a tensile strength of 0.3 to 1.5 kg/cm² depending on the thickness of the non-woven fabric and an adhesion strength of 0.9 to 2.7 kPa.

The hyaluronate non-woven fabric of the present invention has excellent adhesion and tensile strength. Therefore, the hyaluronate non-woven fabric can be used as a mask pack for external skin applications, a biomaterial for tissue repairing, and an anti-adhesion coating material.

### Mode for Invention

Hereinafter, the present invention will be described in more detail with reference to examples. The examples are presented only for illustrative purposes, and it will be apparent to the ordinarily skilled in the art that the scope of the present invention is not to be construed as being limited by the examples.

### Example 1: Preparation of hyaluronate paste

Water was added to sodium hyaluronate having a molecular weight of 150 kDa (Hi-Aqua^{™}, manufactured Jinwoo Bio Co., Ltd.), and then mixed and kneaded to prepare a hyaluronate paste having a moisture content of 50 to 95 wt%. The prepared hyaluronate paste was refrigerated at 4°C for 12 hours to achieve moisture equilibrium.

### Example 2: Manufacture of hyaluronate fiber by spinning

The hyaluronate paste prepared in Example 1 was placed in a storage unit of a spinning device, melted at a temperature in a range of 15°C to 100°C, pressurized to be spun through a nozzle, and dried at 120°C to produce hyaluronate fibers having a moisture content of 5 to 25 wt%.

### Comparative Example 1: Manufacture of hyaluronate fiber by spinning

The hyaluronate paste prepared in Example 1 was placed in a storage unit of a spinning device, melted at a temperature in a range of 15°C to 100°C, pressurized to be spun through a nozzle, and dried at 59°C to produce hyaluronate fibers having a moisture content of 5 to 25 wt%.

### Comparative Example 2: Manufacture of hyaluronate fiber by spinning

The hyaluronate paste prepared in Example 1 was placed in a storage unit of a spinning device, melted at a temperature in a range of 15°C to 100°C, pressurized to be spun through a nozzle, and dried at 160°C to produce hyaluronate fibers.

### Experimental Example 1: Evaluation of roughness of hyaluronate fiber according to drying temperature

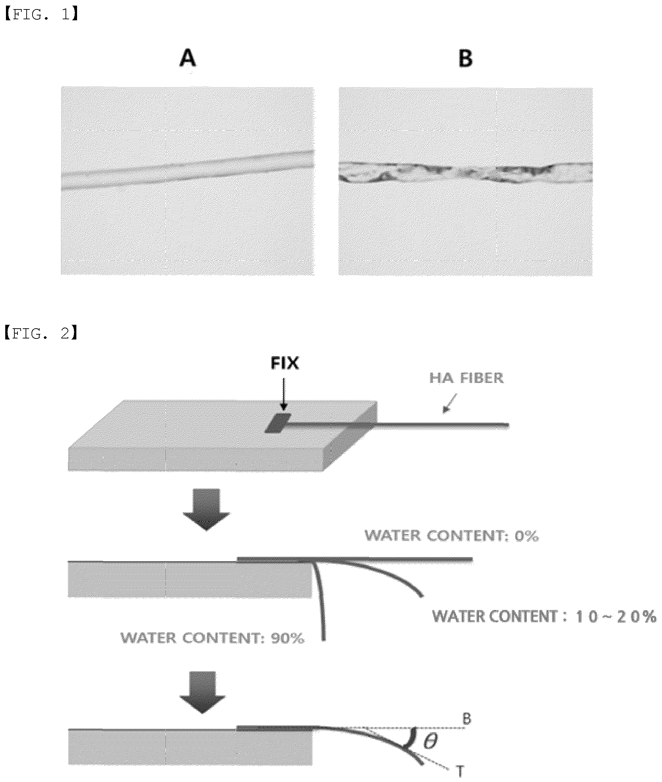

The surface roughness of each of the fibers prepared in Example 2, Comparative Example 1, and Comparative Example 2 was evaluated by microscopy (FIG. 1).

As a result, fibers having a moisture content of 5 to 25 wt% were dried 50°C (Comparative Example 1) and 120°C (Example 2) during spinning. The fibers dried at 50°C during spinning had a smooth surface, but the fibers dried at 120°C during spinning had a rough surface. In the case of Comparative Example 2 in which the drying temperature was 160°C, it was confirmed that the fibers had a moisture content of 1 to 5 wt% and suffered severe browning and cracking.

### Experimental Example 2: Measurement of bending angle of hyaluronate fiber

The bending angle of the hyaluronate fibers prepared in Example 2 was measured by the following method (see FIG. 2), and the results are shown in Table 1.
(a) Prepare HA fibers with a length of 20 cm and a diameter of 1 mm
(b) Fix a first end of each HA fiber by a length of 3 cm on an upper surface of a flat plate
(c) Measure an angle (θ) between an extension line (T) from a second length (remaining end) of each HA fiber and an extension line (B) of the upper surface of the flat plate

**[Table 1]**

| Hyaluronate fiber moisture content (wt%) | Bending angle (°) |
|---|---|
| 50 | 80 |
| 25 | 10 |
| 20 | 8 |
| 10 | 3 |
| 5 | 2 |

From Table 1, it was found that when the moisture content of the hyaluronate fibers exceeded 25 wt%, the bending angle exceeded 10°. In that case, it was not easy to form a web or manufacture a non-woven fabric through needle punching. Even though a non-woven fabric could be manufactured through needle punching, there was a problem in commercialization of the fabric because the fiber and fabric could not withstand during blanking due to their poor physical properties.

In addition, when the moisture content of the hyaluronate fibers was less than 5 wt%, the shape of the fibers could be maintained, but the fibers easily broke because the fibers were not flexible due to their excessive hardness.

On the other hand, when the moisture content of the hyaluronate fibers was in the range of 5 to 25 wt%, the fibers had a bending angle in the range of 2° to 10°, so that the hyaluronate fibers were suitable for the manufacture of non-woven fabric through needle punching.

### Experimental Example 3: Measurement of tensile strength of hyaluronate fiber

The tensile strength of the hyaluronate fibers prepared in Example 2 was measured, and the results are shown in Table 2. The tensile strength was measured using a universal material tester by mounting a sample with a length of 5 cm on a test jig and setting a cross head speed to 2 mm/min.

**[Table 2]**

| Hyaluronate fiber moisture content (wt%) | Tensile strength (N/mm²) |
|---|---|
| 50 | 0.27 |
| 30 | 2.83 |
| 25 | 3.24 |
| 20 | 5.29 |
| 10 | 10.53 |
| 5 | 13.38 |

From Table 2, it was found that when the moisture content of the hyaluronate fibers was 50 wt% or more, the tensile strength of the hyaluronate fibers decreased sharply according to the moisture content, and the fibers easily broke and were not sufficient in elasticity. That is, the fibers were determined to exhibit inferior performance.

In addition, when the moisture content of the hyaluronate fibers was 5 wt% or less, the shape of the fibers was maintained, but the fibers easily broke. Therefore, it was difficult to measure the tensile strength of the fibers.

On the other hand, when the moisture content of the hyaluronate fibers was in the range of 5 to 25 wt%, the tensile strength of the hyaluronate fibers was in the range of 3.24 to 13.38 kg/cm³. Therefore, it was found that yarns made from the hyaluronate fibers could maintain their shape and perform their function.

### Example 3: Manufacture of hyaluronate non-woven fabric through needle punching

### 1) Manufacture of hyaluronate fiber

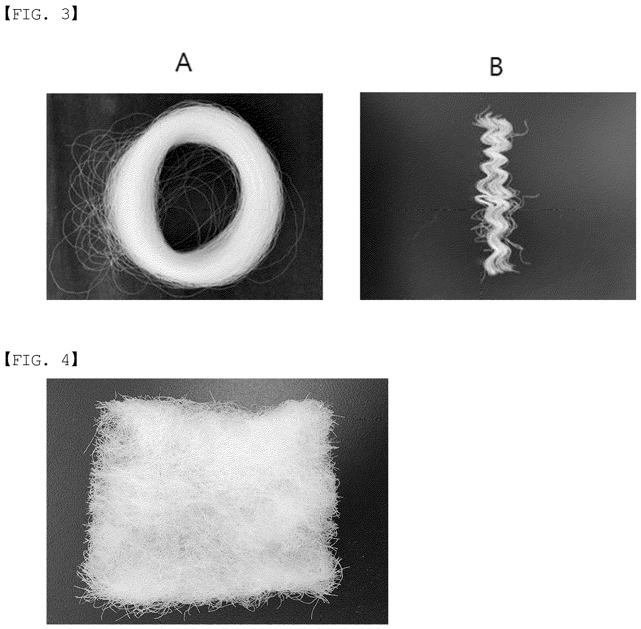

Hyaluronate fibers were manufactured using the method described in Example 2. The hyaluronate fibers had a bending angle of 2° to 10°, a moisture content of 5 to 25 wt%, and a hyaluronate content of 90 wt% or more with respect to the total fiber weight excluding the weight of moisture (see FIG. 3A). The hyaluronate fibers had a tensile strength of 3 to 15 kg/cm³.

### 2) Curling treatment

The manufactured hyaluronate fibers were treated with hot air at 70°C to 100°C to produce curled hyaluronate fibers (see FIG. 3B).

### 3) Cutting

The curled hyaluronate fibers were cut into fiber pieces with a predetermined length of 3 to 7 cm using a cutter.

### 4) Web formation by carding and air-laying

The cut hyaluronate fibers were dispersed by blowing air and then injected into an air stream to form a web.

### 5) Needle punching

Needle punching was performed on multiple layers of webs to produce an even non-woven fabric (with an average thickness of 5 mm) (see FIG. 4).

### Comparative Example 3: Manufacture of hyaluronate non-woven fabric through needle punching

Hyaluronate fibers were manufactured using the method described in Comparative Example 1. The hyaluronate fibers had a bending angle of 2° to 10°, a moisture content of 5 to 25 wt%, and a hyaluronate content of 90 wt% or more with respect to the total fiber weight excluding the weight of moisture. The hyaluronate fibers had a tensile strength of 3 to 15 kg/cm³. Next, a hyaluronate non-woven fabric was manufactured from the hyaluronate fibers using the same method as in Example 3.

As a result, when the hyaluronate fibers with a smooth surface was used, it was confirmed that the fibers were not well tangled during the needle punching process and thus it was difficult to form the non-woven fabric.

### Example 4: Manufacture of hyaluronate and chitosan blended non-woven fabric through needle punching

### 1) Manufacture of hyaluronate fiber

Hyaluronate fibers were manufactured using the same method as in Example 2. The hyaluronate fibers had a bending angle of 2° to 10°, a moisture content of 5 to 25 wt%, and a hyaluronate content of 90 wt% or more with respect to the total fiber weight excluding the weight of moisture. The hyaluronate fibers had a tensile strength of 3 to 15 kg/cm³.

### 2) Chitosan fiber

Manufacturer: Saturn Biotech Co., Ltd., diameter: 0.006 to 0.018 mm

### 3) Curling treatment

Hyaluronate fibers with curl were prepared by hot air treatment at a high temperature in the range of 70°C to 100°C.

Chitosan fibers with curl were prepared by heat-treating chitosan fibers with water vapor having a temperature 100°C or above or by the same hot air treatment process used for the heat treatment of the hyaluronate fibers.

### 4) Cutting

The curled hyaluronate fibers and the curled chitosan fibers were cut into fiber pieces with a predetermined length of 3 to 7 cm using a cutter.

### 5) Web formation by carding and air-laying, and needle punching

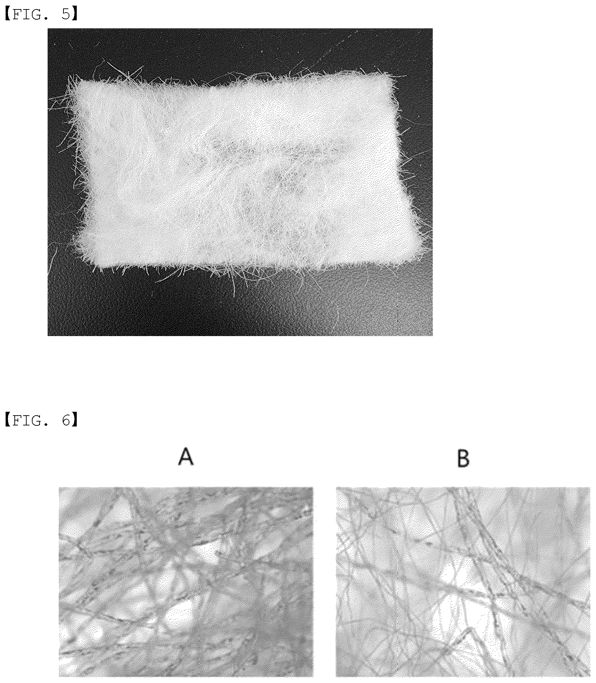

The cut hyaluronate fibers and the cut chitosan fibers were mixed in a 50:50 wt% ratio and treated in the same manner as in Example 3 to produce a hyaluronate and chitosan blended non-woven fabric (average thickness of 5 mm) (see FIG. 5).

### Experimental Example 4: Observation of microstructure of non-woven fabric

A microscope (CX33RTFS2, Olympus Corporation., Korea) was used to observe the microstructure of the hyaluronate non-woven fabrics prepared in Examples 3 and 4 (see FIG. 6). It was possible to identify the web-shaped non-woven structure in which the fibers were cross-lined to each other. Especially for the non-woven fabric in which chitosan was mixed, it was confirmed that the hyaluronate fibers and the chitosan fibers were well mixed. Web-shaped non-woven fabrics have better physical properties than wet spun non-woven fabrics because the web-shaped non-woven fabrics have a space between the cross-linked fibers. For this reason, the web-shaped non-woven fabrics can be easily processed and conveniently used.

### Experimental Example 5: Measurement of gelling degree of hyaluronate non-woven fabric

The gelling degree of each of the pure hyaluronate non-woven fabric manufactured through needle punching (as in Example 3) and the non-woven fabric containing 50 wt% of hyaluronate fibers and 50 wt% of chitosan fibers (as in Example 4) was measured in a manner described below.

The pure hyaluronate non-woven fabric with a size of 2 x 2 cm and the hyaluronate (50%) and chitosan (50%) blended non-woven fabric with the same size were immersed in purified water for 5 seconds and then taken out. Next, water on the surface of each of the non-woven fabrics was removed, and the weight of each fabric was measured to determine the gelling degree. The gelling degree was calculated as follows: Gelling degree (%) = [(weight after gelling - weight before gelling)/weight before gelling] X 100

**[Table 3]**

| Sample | Weight before gelling (g) | Weight after gelling (g) | Gelling degree (%) |
|---|---|---|---|
| Moisture content 25 wt% Pure hyaluronate non-woven fabric | 0.280 | 1.03 | 270 |
| Moisture content 20 wt% Pure hyaluronate non-woven fabric | 0.275 | 1.07 | 290 |
| Moisture content 5 wt% Pure hyaluronate non-woven fabric | 0.255 | 1.14 | 350 |
| Moisture content 25 wt% hyaluronate + chitosan non-woven fabric | 0.398 | 1.07 | 170 |
| Moisture content 20 wt% hyaluronate + chitosan non-woven fabric | 0.386 | 1.11 | 190 |
| Moisture content 5 wt% hyaluronate + chitosan non-woven fabric | 0.370 | 1.25 | 240 |

The chitosan-containing non-woven fabric had a low solubility in water, and it was found that the chitosan-containing non-woven fabric exhibits a lower gelling degree than the pure hyaluronate non-woven fabric.

### Experimental Example 6: Adhesion test of hyaluronate non-woven fabric

Using a texture analyzer (TA) (QM100SN, manufactured by Qmesys co. Ltd., Korea), the adhesion of the pure hyaluronate non-woven fabric (Example 3, pure hyaluronate non-woven fabric with a moisture content of 25 wt%) manufactured through needle punching and the adhesion of the hyaluronate and chitosan 50%:50% blended non-woven fabric (Example 4, hyaluronate plus chitosan non-woven fabric (50%:50% weight ratio) with a moisture content of 25 wt%) were measured as follows:

After cutting the pig skin into 3 cm x 3 cm rectangular fragments, the fragments were immersed in room temperature water for 24 hours so that the moisture content of the fragments became 22.3%. Next, the fragments were attached to upper and lower probes of the texture analyzer, and then each of a pure hyaluronate non-woven fabric, a hyaluronate plus chitosan non-woven fabric, and a pure chitosan non-woven fabric, each having a rectangular shape with a size of 2 cm x 2 cm, was placed on the pig skin on the lower probe, pressed at 3.2 kPa for 3 minutes, and detached at a speed of 10 mm/min to measure the adhesion strength.

**[Table 4]**

| Sample | Adhesion strength (kPa) |
|---|---|
| Pure hyaluronate non-woven fabric | 2.7 |
| Hyaluronate plus chitosan non-woven fabric | 0.9 |
| Chitosan non-woven fabric | 0 |

In the case of the pure chitosan non-woven fabric, it was confirmed that the adhesion strength was zero. In the case of the pure hyaluronate non-woven fabric, it was confirmed that it had a higher adhesion strength than the chitosan-containing non-woven fabric due to its higher gelling degree.

In addition, it was found that the chitosan-containing non-woven exhibited a certain level of adhesion strength.

Through this test, it was found that it was possible to manufacture non-woven fabrics with good physical properties by mixing appropriate amounts of hyaluronate and chitosan, it was possible to control persistence in the body by adjusting the degree of gelling and to provide targeting ability by imparting adhesion.

### Experimental Example 7: Measurement of tensile strength of hyaluronate non-woven fabric

The tensile strength of the pure hyaluronate non-woven fabric (Example 3, pure hyaluronate non-woven fabric with a moisture content of 25 wt%) manufactured through needle punching and the tensile strength of the hyaluronate and chitosan 50%:50% blended non-woven fabric (Example 4, hyaluronate plus chitosan non-woven fabric (50%:50% weight ratio) with a moisture content of 25 wt%) were measured. The results are shown in Table 5. The tensile strength was measured using a universal material tester by mounting a sample with a length of 5 cm on a test jig and setting a crosshead speed to 2 mm/min.

**[Table 5]**

| Sample | Tensile strength (kgf) |
|---|---|
| Pure hyaluronate non-woven fabric | 0.3 |
| Hyaluronate plus chitosan non-woven fabric | 1.5 |

From Table 5, it was found that when chitosan fibers were mixed into pure hyaluronate fibers, the non-woven fabric manufactured from the blended fibers exhibited a higher tensile strength. When chitosan fibers were added when forming non-woven fabrics, it was found that chitosan fibers were tangled between hyaluronate fibers of the non-woven fabrics, so that the binding force was increased.

Specific parts of the present invention have been described in detail, and those who ordinarily skilled in the art will appreciate that the specific parts described are only for illustrative purposes and the scope of the present invention is not limited by the specific parts described above. Thus the substantial scope of the present invention will be defined by the appended claims and their equivalents.

### Industrial Applicability

The hyaluronate non-woven fabric of the present invention has excellent adhesion and tensile strength. Therefore, the hyaluronate non-woven fabric of the present invention can be used as a mask pack for external skin applications, a biomaterial for tissue repairing, and an anti-adhesion coating material.

## Claims

1. A hyaluronate non-woven fabric comprising hyaluronate fibers, the fibers having a bending angle of 2° to 10°, a moisture content of 5 to 25 wt%, and a hyaluronate content of 90 wt% or more with respect to a total fiber weight excluding a weight of moisture, as measured by a following described below:
<Bending Angle Measurement Method>
(a) Prepare HA fibers with a length of 20 cm and a diameter of 1 mm;
(b) Fix a first end of each HA fiber by a length of 3 cm on an upper surface of a flat plate; and
(c) Measure an angle (θ) between an extension line (T) from a second length (remaining end) of each HA fiber and an extension line (B) of the upper surface of the flat plate.

2. The hyaluronate non-woven fabric of claim 1, wherein the hyaluronate fiber has a tensile strength of 3 to 15 kg/cm².

3. The hyaluronate non-woven fabric of claim 1, wherein the hyaluronate non-woven fabric has an adhesion strength in a range of 0.9 to 2.7 kPa.

4. The hyaluronate non-woven fabric of claim 1, wherein the hyaluronate non-woven fabric has a thickness in a range of 1 to 10 mm.

5. The hyaluronate non-woven fabric of claim 1, wherein the hyaluronate fabric has a tensile strength in a range of 0.3 to 1.5 kg/cm².

6. The hyaluronate non-woven of claim 1, wherein the hyaluronate non-woven fabric is for use as a mask pack for external application to the skin, a biomaterial for tissue repairing, or an anti-adhesion coating material.

7. A method of manufacturing a hyaluronate non-woven fabric, the method comprising:
(a) preparing hyaluronate fibers;
(b) treating the hyaluronate fibers to be curled;
(c) cutting the curled hyaluronate fibers;
(d) forming a web with the cut hyaluronate fibers; and
(e) needle punching the hyaluronate web.

8. The method of claim 7, wherein the preparing of the hyaluronate fibers comprises:
(a-1) preparing a hyaluronate paste by adjusting the moisture content of a hyaluronate having an average molecular weight of 100 to 3,000 kDa to a range of 50 to 95 wt%;
(a-2) melting the moisture-controlled hyaluronate paste at a temperature in a range of 15°C to 100°C and extruding a fluid resulting from the melting of the hyaluronate paste through a nozzle; and
(a-3) drying the extruded spun fluid to form fibers.

9. The method of claim 7, wherein the prepared hyaluronate fibers have a tensile strength of 3 to 15 kg/cm².

10. The method of claim 7, wherein when forming the web, fibers made of a polymeric material selected from the group consisting of collagen, fibrin, gelatin, chitosan, alginic acid, polylactic acid (PLA), polyglutamic acid (PGA), polylactide-glycolide copolymer (PLGA), and polycaprolactone (PCL) is added and mixed.
